# EUROPEAN PATENT SPECIFICATION

(11) **EP 1 610 771 B1**
(45) Date of publication and mention of the grant of the patent: **03.12.2008**
(21) Application number: 04726526.9
(22) Date of filing: 08.04.2004
(51) Int. Cl.: A61K 31/00, A61K 31/4164, A61K 31/4178, A61P 25/08

(54) **USE OF AN ALPHA2-ADRENOCEPTOR ANTAGONIST FOR THE TREATMENT OF EPILEPSY**
VERWENDUNG VON ALPHA2-ADRENOREZEPTOR ANTAGONISTEN ZUR BEHANDLUNG VON EPILEPSIE
UTILISATION D'UN ANTAGONISTE DE L'ADRENORECEPTEUR ALPHA-2 POUR LE TRAITEMENT DE L'EPILEPSIE

(30) Priority: 10.04.2003 US 461413 P
(43) Date of publication of application: 04.01.2006
(73) Proprietor: ORION CORPORATION, 02200 Espoo (FI)
(72) Inventor: HAAPALINNA, Antti, FI-20360 Turku (FI); PITKÄNEN, Asla, FI-70700 Kuopio (FI)
(74) Representative: Sexton, Jane Helen
(86) International application number: PCT/FI2004/000220
(87) International publication number: WO 2004/089349

(56) References cited:
- EP-A- 0 194 984
- EP-A- 0 304 910
- WO-A-03/082825
- PITKANEN ASLA ET AL: "Atipamezole, an alpha2-adrenoceptor antagonist, has disease-modifying effects on epileptogenesis in rats." EPILEPSIA, vol. 44, no. Supplement 9, 2003, pages 261-262, XP001193760 & ANNUAL MEETING OF THE AMERICAN EPILEPSY SOCIETY; BOSTON, MA, USA; DECEMBER 05-10, 2003 ISSN: 0013-9580
- PITKANEN A (REPRINT) ET AL: "Disease modifying effects of alpha2-adrenoceptor blocker, atipamezole , on epileptogenesis in rats" EPILEPSIA, (FEB 2003) VOL. 44, SUPP. [8], PP. 175-175. PUBLISHER: BLACKWELL PUBLISHING INC, 350 MAIN ST, MALDEN, MA 02148 USA. ISSN: 0013-9580., February 2003 (2003-02), XP008031700
- MIRSKI MAREK A Z ET AL: "Dexmedetomidine decreases seizure threshold in a rat model of experimental generalized epilepsy" ANESTHESIOLOGY (HAGERSTOWN), vol. 81, no. 6, 1994, pages 1422-1428, XP008031693 ISSN: 0003-3022
- VALTONEN, PIRJO ET AL: "Effect of .alpha.2-adrenergic drugs dexmedetomidine and atipamezole on extracellular amino acid levels in vivo" EUROPEAN JOURNAL OF PHARMACOLOGY , 285(3), 239-46 CODEN: EJPHAZ; ISSN: 0014-2999, 1995, XP001193834
- HALONEN, TOIVO ET AL: ".alpha.2-Adrenoceptor agonist, dexmedetomidine, protects against kainic acid-induced convulsions and neuronal damage" BRAIN RESEARCH , 693(1,2), 217-24 CODEN: BRREAP; ISSN: 0006-8993, 1995, XP001193835 cited in the application

## Description

### FIELD OF THE INVENTION

The present invention relates to a medicament for inhibiting the development of epilepsy. One embodiment of the present invention relates, for instance, to a medicament for the inhibition of the development of epilepsy which comprises an alpha2-adrenoceptor antagonist, such as defined in claim 1.

### BACKGROUND OF THE INVENTION

Symptomatic partial epilepsy often develops in 3 phases: an initial brain damaging insult like status epilepticus (SE), head trauma, stroke, infection, neurosurgical operation 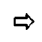 latency phase (epileptogenesis) 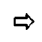 spontaneous seizures (epilepsy). The current treatment of epilepsy exclusively focuses on prevention or suppression of seizures. Thus, there is an unmet need for a pharmaceutical capable of inhibiting the development of epilepsy in the patients at risk. Further, if epilepsy can not be completely prevented, it would be beneficial to modify the disease process in such a way that epileptogenesis leads to a milder and easier-to-control disease.

Previous studies have convincingly demonstrated that α2-adrenergic agonists are anticonvulsant in acute seizure models, including chemically induced seizures with kainic acid (Baran H. et al., Eur J Pharmacol 1985:113:263-269) or pentylenetetrazol (Kulkarni SK., Arch Int Pharmacodyn 1981:252:124-132) as well as audiogenic seizures in genetically epilepsy prone DBA/2J mice (Kellog C., J Neurochem 1976:106:87-103). On the other hand, studies with nonspecific α2-adrenoceptor antagonists show that they are proconvulsants in rats (Gellmann RL. et al., J Pharmacol Exp Ther 1987:241(3):891-898, Janumpalli S. et al., J Neurosci 1998:18(6):2004-2008). In humans, oral administration of an α2-adrenoceptor antagonist, yohimbine, increased cortical excitability induced by transcranial magnetic stimulation (Plewnia C et al., Neurosci Lett 2001:307:41-44).

Atipamezole is a specific alpha₂- adrenoceptor antagonist that has a high alpha₂/alpha₁- selectivity ratio and does not display a species differences (human and rodent) in its effects. Like other α2-adrenoceptor antagonists, acute administration of atipamezole has a proconvulsant effect of kainate-induced seizures (Halonen T. et al., Brain Res 1995:693:217-224).

### DETAILED DESCRIPTION OF THE INVENTION

Applicants have surprisingly discovered that a selective alpha2-adrenoceptor antagonist, atipamezole (4-(2-ethyl-2,3-dihydro-1H-inden-2-yl)-1H-imidazole hydrochloride), has a clear disease-modifying effect on epileptogenesis, that is, epilepsy that develops is milder and can be non-progressive. Thus, selective alpha2-adrenoceptor antagonists, such as atipamezole, and their pharmacologically acceptable salts, can be used for the inhibition of the development of epilepsy.

Alpha2-adrenoceptor antagonists of the invention are 4-(2-ethyl-2,3-dihydro-1H-inden-2-yl)-1H-imidazole, known as atipamezole, and its pharmaceutically acceptable acid addition salts with inorganic and organic acids generally used for the purpose, are described in U.S. Patent. No. 4,689,339, and the halogenated analogs of atipamezole which are 4-(2-ethyl-5-fluoro-2,3-dihydro-1H-inden-2-yl)-1H-imidazole and 4-(2-ethyl-5,6-difluoro-2,3-dihydro-1H-inden-2-yl)-1H-imidazole and their pharmaceutically acceptable acid addition salts have been described in U.S. Patent No. 5,498,623.

For the purposes of this disclosure the term "selective alpha2-adrenoceptor antagonist" refers to a compound having an alpha2/alphal ratio greater than about 30.

The precise amount of the drug to be administered to a mammal according to the present invention is dependent on numerous factors known to one skilled in the art, such as the compound to be administered, the general condition of the patient, the condition to be treated, the desired duration of use, the type of mammal, the method and route of administration. For example, for atipamezole, the usual daily dosage can be from 1 to 50 mg for a 70 kg person, and can be from 10 to 30 mg, for instance, divided in 1 to 4 individual doses. In another embodiment, the dose for atipamezole can be about 10 mg. Typical routes of administration include, oral, transdermal, transmucosal, and parenteral routes.

The use of the alpha2-adrenoceptor antagonist can be started, for example, one or more days after an initial brain damaging insult like head trauma, brain ischemia, infection or neurosurgical operation, and it can be started even several weeks after such insult. Furthermore, it can be started during the interictal period (i.e. a period between two seizures) following the initial brain damaging insult. Depending on the severity of the initial brain damaging insult and the general condition of the patient, the treatment can be continued for from several weeks to several months and even years.

The invention will be further illustrated by the following example.

### EXAMPLE 1

To mimic the clinical environment, α2-adrenoceptor antagonist treatment with atipamezole was started 1 wk after induction of status epilepticus (SE) with subcutaneous Alzet minipumps and continued for 9 wk. Two independent studies were carried out.

### Animals

Male Harlan Sprague-Dawley rats (300-350 g; Netherlands) were used in this study. After implantation of electrodes, rats were housed in individual cages at a temperature of 19-21 °C, with humidity at 50-60% and lights on 7.00-19.00. Standard food pellets and water were freely available.

### Implantation of electrodes

The animals were anaesthetized with an intraperitoneal injection of sodium pentobarbital (60 mg/kg) and chloral hydrate (60 mg/kg) and inserted into a stereotaxic frame (lambda and bregma at the same horizontal level). A pair of stimulating electrodes (0.5 mm tip separation) was implanted into the lateral nucleus of the left amygdala (3.6 mm posterior, 5.0 mm lateral, and 6.5 mm ventral to bregma). One stainless steel screw was inserted into the skull above the right frontal cortex and served as cortical electrode (3 mm anterior to the bregma, 2 mm lateral to midline). Two stainless steel screws were inserted into the skull bilaterally over the cerebellum, which served as indifferent and ground electrodes. The electrodes were fixed with dental acrylate. The rats were allowed to recover from surgical operation for 14 d, after which electrical stimulation was initiated.

### Induction of SE

After discharge threshold was assessed by stimulating the amygdala with a one-second train of one-msec bipolar square-wave pulses (60 Hz, 50-400 µA peak to peak). Only those rats in which after discharges could be induced at 400 µA or at lower current level were included in the study.

To induce SE, the amygdala was stimulated repeatedly at 500 msec intervals with a train of pulses lasting 100 msec (1 msec 60 Hz bipolar pulses at 400 µA peak to peak). After 20 minutes, stimulation was interrupted, and the behavioral and electrographic seizure activity was observed for 5 min. If the animal did not meet the criterion of clonic SE (continuous epileptiform spiking and recurrent clonic seizures), stimulation was resumed and the behavior of the animal was checked again after 10 min. Once the criterion of SE was achieved, no further stimulation was given. After 3 h, SE was terminated by injecting diazepam 20 mg/kg (i.p.).

### Administration of vehicle, diazepam, and atipamezole

Vehicle (0.9% saline, Natrosteril 9mg/ml, Baxter, Vantaa, Finland) was administered via subcutaneous Alzet minipumps with a pumping rate of 9.15 µl/h in 1-wk and 2.61 µl/h in 8-wk pumps starting 1 wk after stimulation. Minipumps were implanted subcutaneously to the back between the scapulae according to the instructions provided by manufacturer. For implantation, animals were anesthetized with an intraperitoneal injection of sodium pentobarbital (60 mg/kg) and chloral hydrate (60 mg/kg).

Diazepam was administered at 3 h after the beginning of SE (20 mg/kg, i.p.). Atipamezole treatment was started 1 wk after SE. Compound was administered with subcutaneous Alzet osmotic minipumps and continued for 9 wk. Atipamezole was dissolved into sterile 0.9 % NaCl. The concentration of atipamezole was adjusted according to the weight of the rat and pumping rate to obtain a constant flow-rate of 100µg/kg/h.

### Study Groups

Treatment groups in Study 1:
■ DZP (diazepam) + vehicle (n=11): Rats with SE treated with DZP (20 mg/kg, i.p.) at 3 h after the beginning of SE. Chronic treatment with vehicle (0.9% NaCl, subcutaneous Alzet minipumps) for 9 wk starting 1 wk after SE.
■ DZP+ atipamezole (n=7): Rats with SE treated with DZP (20 mg/kg, i.p.) at 3 h after the beginning of SE. Chronic treatment with atipamezole (100µg/kg/h, subcutaneous Alzet minipumps) for 9 wk starting 1 wk after SE.

Treatment groups in Study 2:
■ Controls (n=9): Operated, non-stimulated and non-treated controls.
■ ∅ + vehicle (n=11): No treatment at 3 h after the beginning of SE. Chronic treatment with vehicle (0.9% NaCl in subcutaneous Alzet minipump) for 9 wk starting 1 wk after SE.
■ DZP + vehicle (n=10): Diazepam treatment (20 mg/kg, i.p.) at 3 h after the beginning of SE. Chronic treatment with vehicle (0.9% NaCl in subcutaneous Alzet minipump) for 9 wk starting 1 wk after SE.
■ ∅ + atipamezole (n=10). No treatment at 3 h after the beginning of SE. Chronic treatment with atipamezole (100µg/kg/h, subcutaneous Alzet minipumps) for 9 wk starting 1 wk after SE.
■ DZP + atipamezole (n=12). Diazepam treatment (20 mg/kg, i.p.) at 3 h after the beginning of SE. Chronic treatment with atipamezole (100µg/kg/h, subcutaneous Alzet minipumps) for 9 wk starting 1 wk after SE.

### RESULTS

The data combined from the two different studies is summarized in Table 1. None of the animals in the DZP+atipamezole group developed severe epilepsy, whereas 50% the rats in the DZF+vehicle group had >1 seizure/day (p>0.05). No progression was observed between the two follow-ups.

**Table 1. Number of animals with severe (>1 seizure/day) or mild (≤1 seizure/day) epilepsy in the different treatment groups.**

| | **During treatment** | | **After treatment** | | **All** | |
|---|---|---|---|---|---|---|
| | severe | mild | severe | mild | severe | mild |
| **Study 1** | | | | | | |
| DZP + vehicle | 4/10 | 6/10 | 4/9 | 5/9 | 5/10 | 5/10 |
| DZP + atipamezole | 0/3 | 3/3 | 0/4 | 4/4 | 0/5 | 5/5 |
| *Fischer's exact test* | ns | | ns | | ns | |
| | | | | | | |

| **Study 2** | | | | | | |
|---|---|---|---|---|---|---|
| ∅ + vehicle | 3/8 | 5/8 | 3/7 | 4/7 | 4/8 | 4/8 |
| DZP + vehicle | 3/4 | 1/4 | 2/3 | 1/3 | 2/4 | 2/4 |
| ∅ + atipamezole | 1/8 | 7/8 | 1/7 | 6/7 | 1/8 | 7/8 |
| DZP + atipamezole | 0/9* | 9/9 | 0/9* | 9/9 | 0/9 | 9/9# |
| *Fischer's exact test* | p<0.05 | | p<0.05 | | p<0.05 | |
| | | | | | | |

| **Study 1** + **Study 2** | | | | | | |
|---|---|---|---|---|---|---|
| Ø/DZP + vehicle | 10/22 | 12/22 | 10/19 | 9/19 | 11/22 | 11/22 |
| ∅/DZP + atipamezole | 1/20 | 19/20 | 1/20 | 19/20 | 1/22 | 21/22 |
| *Fischer's exact test* | p<0.01 | | p<0.01 | | p<0.01 | |

| | | | | | | |
|---|---|---|---|---|---|---|
| Abbreviations: ns, nonsignificant. Statistical significances between the atipamezole treated and untreated animals: * p<0.05 compared to the DZP+vehicle group; # p<0.05 compared to the ∅+vehicle group (Fischer's exact test ) | | | | | | |

## Claims

1. An alpha2-adrenoceptor antagonist for use in inhibiting the development of epilepsy in a patient at risk of developing epilepsy, wherein the alpha2-adrenoceptor antagonist is atipamezole, or a halogenated analog of atipamezole, which is 4-(2-ethyl-5-fluoro-2,3-dihydro-1H-inden-2-yl)-1H-imidazole or 4-(2-ethyl-5,6-difluoro-2,3-dihydro-1H-inden-2-yl)-1H-imidazole, or a pharmaceutically acceptable salt thereof.

2. The alpha2-adrenoceptor antagonist as claimed in claim 1, which is atipamezole or a pharmaceutically acceptable salt thereof.

3. The alpha2-adrenoceptor antagonist as claimed in claim 1, which is 4-(2-ethyl-5-fluoro-2,3-dihydro-1H-inden-2-yl)-1H-imadazole or a pharmaceutically acceptable salt thereof.

4. The alpha2-adrenoceptor antagonist as claimed in claim 1 wherein said risk of developing epilepsy is caused by head trauma, brain ischemia, infection or neurosurgical operation.

## Patentansprüche

1. alpha2-Adrenozeptor-Antagonist zur Inhibierung der Entwicklung von Epilepsie bei einem Patienten mit einem Risiko Epilepsie zu entwickeln, wobei der alpha2-Adrenozeptor-Antagonist Atipamezol oder ein halogeniertes Analogon von Atipamezol ist, welches 4-(2-Ethyl-5-fluor-2,3-dihydro-1H-inden-2-yl)-1H-imidazol oder 4-(2-Ethyl-5,6-difluor-2,3-dihydro-1H-inden-2-yl)-1H-imidazol ist, oder ein pharmazeutisch verträgliches Salz davon.

2. alpha2-Adrenozeptor-Antagonist nach Anspruch 1, welcher Atipamezol oder ein pharmazeutisch verträgliches Salz davon ist.

3. alpha2-Adrenozeptor-Antagonist nach Anspruch 1, welcher 4-(2-Ethyl-5-fluor-2,3-dihydro-1H-inden-2-yl)-1H-imidazol oder ein pharmazeutisch verträgliches Salz davon ist.

4. alpha2-Adrenozeptor-Antagonist nach Anspruch 1, wobei das Risiko, Epilepsie zu entwickeln, durch ein Schädeltrauma, eine Hirnischämie, eine Infektion oder eine neurochirurgische Operation verursacht wird.

## Revendications

1. Antagoniste du récepteur alpha2-adrénergique à utiliser dans l'inhibition du développement d'une épilepsie chez un patient à risque de développer une épilepsie, où l'antagoniste du récepteur alpha2-adrénergique est l'atipamézole ou un analogue halogéné de l'atipamézole qui est le 4-(2-éthyl-5-fluoro-2,3-dihydro-1H-indén-2-yl)-1H-imidazole ou le 4-(2-éthyl-5,6-difluoro-2,3-dihydro-1H-indén-2-yl)-1H-imidazole ou un sel pharmaceutiquement acceptable de ceux-ci.

2. Antagoniste du récepteur alpha2-adrénergique selon la revendication 1, qui est l'atipamézole ou un sel pharmaceutiquement acceptable de celui-ci.

3. Antagoniste du récepteur alpha2-adrénergique selon la revendication 1, qui est le 4-(2-éthyl-5-fluoro-2,3-dihydro-1H-indén-2-yl)-1H-imidazole ou un sel pharmaceutiquement acceptable de celui-ci.

4. Antagoniste du récepteur alpha2-adrénergique selon la revendication 1, où ledit risque de développer une épilepsie est provoqué par un traumatisme crânien, une ischémie cérébrale, une infection ou une opération neurochirurgicale.
